# EUROPEAN PATENT APPLICATION

(11) **EP 2 070 894 A1**
(43) Date of publication of application: **17.06.2009**
(21) Application number: 07254818.3
(22) Date of filing: 12.12.2007
(51) Int. Cl.: C07C 1/04, C07C 1/24, C07C 11/09, C07C 2/76, C07C 11/10

(54) **A process for the conversion of n-butanol of n-butanol to di-isobutene and pentene**

(71) Applicant: BP p.l.c., London SW1Y 4PD (GB)
(72) Inventor: The designation of the inventor has not yet been filed
(74) Representative: De Kezel, Eric

(57) **Abstract**

The present invention relates to a process for the conversion of n-butanol to di-isobutene and pentene and/or di-pentene, in particular the present invention relates to the conversion of synthesis gas to di-isobutene and pentene and/or di-pentene.

## Description

The present invention relates to a process for the conversion of n-butanol to isobutene and pentene. In particular, the present invention relates to a process for the conversion of n-butanol to di-isobutene alongside the conversion of n-butanol to pentene and/or di-pentene. More particularly, the present invention relates to the conversion of synthesis gas to di-isobutene alongside the conversion of synthesis gas to propene and/or di-pentene.

Pentene and di-pentene are highly desirable products and are of major commercial importance.

Di-isobutene is also a highly desirable product and is of major commercial importance; since it may be hydrogenised to make iso-octane, which is a major gasoline component for high octane fuels.

Hence, there has been much commercial interest in the production of di-isobutene. Typically, the favoured route has been to use iso-butene to produce di-isobutene, and hence there are many methods employed for its production, these include the following.

KR20040111120 provides a process for preparing isobutene, which is satisfactory in terms of selectivity, space-time yield, energy consumption and/or capital costs by using a dissociation reactor which is operated isothermally or at least pseudo-isothermally. This process for preparing isobutene comprises dissociating tertiary butanol into isobutene and water via a strong acid ion-exchange resin arranged as a fixed bed in at least one reactor at a temperature of from 80 to 150 deg.C and a pressure of from 5 to 25 bar to obtain a reaction mixture and separating the reaction mixture into isobutene, a by-product, water and at least one mixture of undissociated tertiary butanol and water; wherein the reactor is operated pseudo-isothermally, with a temperature difference between inflowing and outflowing streams of less than 15 K. In the process, a feed to the reactor comprises a fresh feed stream and at least one recycled streams containing a mixture of undissociated t-butanol, water and optionally the by-product, wherein a mass ratio of the fresh feed stream to the sum of the recycled streams is less than 1:10.

KR880002271B describes a process consisting of (i) continuously supplying an aqueous solution containing about 40-90 wt% of TBA into rxn. container, (ii) dehydrating as a homogeneous liquid phase at 80-150 deg.C and at 5-25 bar on the fixed-catalyst-bed composed of strong acidic ionexchange resin, (iii) fractionally distg. homogeneous liquid rxn. mixture at distg. parts sepd. from rxn. chamber and separating isobutene from water/unreacted TBA and (iv) concentrating unreacted TBA by distillation and resupplying into rxn. container with circulation rate (in here a mixture of newly supplied TBA and TBA/water mixture contd. about 40-90 wt.% of TBA, with excess water expelled

US2005080305 describes a process for producing di-isobutene from isobutene. The process comprises first oligomerizing isobutene to di-isobutene using a sulfonic acid-type ion exchange resin. The oligomerization step is followed by contacting the di-isobutene product with an adsorbent to remove sulphur impurities produced during the oligomerization step. The adsorbent is a large pore zeolite such as zeolite X and zeolite Y. Optionally, the purified di-isobutene may be hydrogenated to isooctane using a hydrogenation catalyst.

JP54138506 describes a process to obtain highly pure isobutene in high yield, continuously, with little external heat supply and little deterioration of a catalyst, by the decomposition treatment of tert-butanol under specific conditions in the presence of a cation- exchange resin catalyst.

JP54135710 describes a process to obtain isobutene under mild conditions without external heating, in high yield, by the dehydration decomposition of a tertiary butanol (TBA) continuously supplying a gaseous TBA into the reaction vessel containing liquid TBA, water and a catalyst.

EP0485145 describes a process for the production of olefins which comprises passing an oxygenate-containing feedstock over a zeolite catalyst at a temperature greater than 200 DEG C characterised in that the oxygenate-containing feedstock comprises C3 and/or C4 olefins and as oxygenate methanol formaldehyde and/or dimethylether, the molar ratio of olefin to oxygenate being greater than 1:20 and the zeolite type catalyst being of TON-type structure.

It is has long been desired, yet unaccomplished, to produce isobutene (and hence di-isobutene) from a feedstock comprising n-butanol. As shown in the aforementioned prior art, isobutene has typically been produced through the conversion of iso-butanol and/or tertiary butanol. Namely due to the steric form of n-butanol, i.e. n-butanol exists as a linear compound. However the applicants have now found a process for producing isobutene (and therefore di-isobutene) directly from n-butanol.

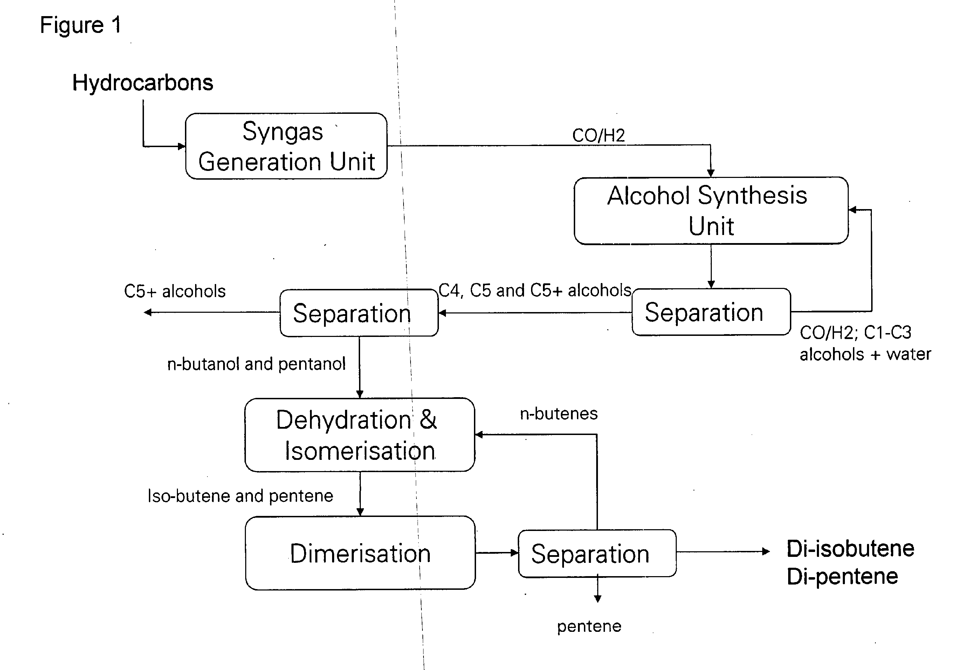

Figure 1 represents an embodiment of a process scheme according to the present invention, wherein the references correspond to those used in the present description and appending claims.

Thus, the present invention relates to a process for the coproduction of pentene and iso-butene by dehydrating and isomerising an n-butanol feedstock in the presence of a crystalline aluminosilicate catalyst. The Applicants have unexpectedly found that the use of the claimed catalyst in the process of the present invention allows to convert n-butanol into both pentene and iso-butene with a yield and selectivity never achieved before.

Furthermore, the present invention relates to a process for producing di-isobutene and pentene and/or di-pentene from n-butanol, characterised by the following consecutive steps:
1) dehydrating and isomerising an n-butanol feedstock in the presence of a crystalline aluminosilicate catalyst to produce an olefin stream comprising pentene, n-butenes and iso-butene,
2) dimerising at least a part of the stream produced in step 1 to produce a stream comprising di-isobutene and unreacted n-butenes,
3) separating and recycling at least a part of the unreacted n-butenes from step 2 to the dehydration and isomerisation step 1,
4) recovering di-pentene from step 2 and/or separating and recovering pentene from either step 1 and/or step 2, and
5) recovering the di-isobutene from step 2.

The present invention also relates to a process for producing di-isobutene and pentene and/or di-pentene from synthesis gas characterised by the following consecutive steps:
1) converting a synthesis gas feed to a mixed alcohol feedstock, containing n-butanol,
2) dehydrating and isomerising a least a part of the n-butanol from step 1, in the presence of a crystalline aluminosilicate catalyst, to produce an olefin stream comprising pentene, n-butenes and iso-butene,
3) dimerising at least a part of the stream produced in step 2 to produce a stream comprising di-isobutene and unreacted n-butenes,
4) separating and recycling at least a part of the unreacted n-butenes from step 3 to the dehydration and isomerisation step 2,
5) recovering di-pentene from step 3 and/or separating and recovering pentene from either step 2 and/or step 3, and
6) recovering the di-isobutene form step 3.

According to one aspect of the present invention, the synthesis gas feed (a mixture of carbon oxide(s) and hydrogen) that is used to produce the mixed alcohol feedstock, is preferably produced from a hydrocarbon feedstock.

The hydrocarbon feedstock used for synthesis gas generation is preferably a carbonaceous material, for example biomass, plastic, naphtha, refinery bottoms, crude synthesis gas (from underground coal gasification or biomass gasification), smelter off gas, municipal waste, coal, and/or natural gas, with coal and natural gas being the preferred sources, and natural gas being the most preferable source.

Natural gas commonly contains a range of hydrocarbons (e.g. C1-C3 alkanes), in which methane predominates. In addition to this, natural gas will usually contain nitrogen, carbon dioxide and sulphur compounds. Preferably the nitrogen content of the feedstock is less than 40 wt %, more preferably less than 10 wt % and most preferably less than 1 wt %.

Processes for producing mixtures of carbon oxide(s) and hydrogen (commonly known as synthesis gas), in a synthesis gas reactor, are well known. Each method has its advantages and disadvantages, and the choice of using a particular reforming process over another is governed by economic and available feed stream considerations, as well as by the desire to obtain the optimum (H2-CO2):(CO+CO2) molar ratio in the resulting synthesis gas that is suitable for further chemical processing. A discussion of the available synthesis gas production technologies is provided in both "Hydrocarbon Processing" V78, N.4, 87-90, 92-93 (April 1999) and "Petrole et Techniques", N. 415, 86-93 (July-August 1998), and are both hereby incorporated by reference.

It is also known that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

Typically, for commercial synthesis gas production the pressure at which the synthesis gas is produced from a steam reformer ranges from approximately 0.1 to 10 MPa, preferably 2.0 to 3.0 MPa and the temperatures at which the synthesis gas exits the reformer ranges from approximately 700°C to 1000°C. Likewise, for commercial synthesis gas production the pressure at which the synthesis gas is produced from an auto-thermal reformer ranges from approximately 0.1 to 10 MPa, preferably 2.0 to 5.0 MPa and the temperatures at which the synthesis gas exits the reformer ranges from approximately 700°C to 1300°C.

The high temperatures are necessary in order to produce a favourable equilibrium for synthesis gas production, and to avoid metallurgy problems associated with carbon dusting. The synthesis gas contains a molar ratio of (H2-CO2):(CO+CO2) ranging from 0.8 to 3.0, which is dependent on the hydrocarbon feedstock(s) and the method of reforming used. For example, when natural gas is used as the hydrocarbon feedstock for steam reforming, the synthesis gas obtained usually has a (H2-CO2):(CO+CO2) maximum ratio of 3.0. However, when natural gas is used as the hydrocarbon feedstock for auto-thermal reforming, the synthesis gas obtained usually has a (H2-CO2):(CO+CO2) ratio of 1.5.

According to a preferred embodiment of this aspect of the present invention, the exit stream obtained from the synthesis gas reactor (e.g. using a steam reformer), comprises essentially a mixture of carbon oxide(s) and hydrogen. It can also comprise water, nitrogen and traces of unconverted hydrocarbon (e.g. C1-C3 alkanes).

According to this aspect of the present invention, said synthesis gas stream is then introduced into an alcohol synthesis unit, in order to produce a stream comprising mixed alcohols.

The alcohol(s) synthesis reaction according to this aspect of the present invention is preferably performed by passing a mixture of hydrogen and carbon monoxide over a conversion catalyst as a vapour phase reaction (e.g. using a fixed bed and/or fluidized bed reactor) or as a liquid phase reaction in an essentially involatile and inert solvent, such as a hydrocarbon (e.g. using a slurry reactor) or in a jet loop reactor.

The said alcohol synthesis reaction is preferably operated at a temperature of more than 190 DEG C and less than 400 DEG C; and a pressure of more than 1.9 MPa and less than 20 MPa. The term alcohol synthesis reactor, as used in the present invention, pertains to any appropriate reactor, e.g. a fixed bed reactor using 80 tonnes of catalyst. The reactants may be fed upwards or downwards to the catalyst, or a combination of both, to a fixed bed located in a multi-tubular reactor or a packed bed arrangement. The reaction may be effected in a dynamic bed of the catalyst. In such a reaction, the bed of catalyst is moving such as in the case of a slurry bed of the catalyst. The alcohol synthesis reactor may preferably be chosen amongst tubular, multitubular, slurry, moving bed, fluidised bed, radial bed, jet loop reactor, multibed or reactive distillation reactor. According to an embodiment of the present invention, a fixed bed reactor is used, preferably a radial bed(s) or a multitubular vapour phase reactor or a combination thereof is used. Most preferably the oxygenate synthesis reactor comprises a series of adiabatic fixed bed reactors operated in either a longitudinal and/or radial flow mode.

The conversion catalyst used in the alcohol synthesis unit is preferably a modified molybdenum sulphide based catalyst, or a modified methanol based catalyst and/or a modified Fischer-Tropsch catalyst and/or a precious metal base catalyst, such as a rhodium based catalyst.

Molybdenum sulphide based catalysts are preferred; these can be modified by a promoter. Promoter(s) can be added as salts during the catalyst preparation, and are preferably potassium ions (e.g. derived from a salt of potassium, such as potassium carbonate or acetate). The preferred loadings of potassium ions per molybdenum is comprised between 0.7 and 1.5, most preferably between 1.0 and 1.4.

The preferred conversion catalyst, according to the present invention, is a molybdenum sulphide based catalysts containing cobalt, the cobalt to molybdenum molar ratio being preferably comprised between 0.5 and 3.0, more preferably between 0.5 and 1.0 and most preferably between 0.5 and 0.9.

According to a preferred embodiment of the present invention, the alcohol(s) targeted during alcohol synthesis are primarily butanol(s) (n-butanol and iso-butanol); however C1 to C3 alcohols and/or C4 + alcohols may also be produced during the alcohol synthesis stage. Said butanol(s) preferably represent together at least 10% preferably more than 15% by carbon content of the products (products defined as being all products exiting the alcohols synthesis unit excluding CO2 and CO) obtained from the alcohol synthesis reactor.

Additionally, oxygenates such as esters, ethers and acids may also be produced as by-products of the reaction.

According to a preferred embodiment of this aspect of the present invention, the said mixed alcohols stream is subjected to at least one separation in order to obtain a stream with a high n-butanols content, known as the n-butanol rich stream (where n-butanol represents at least 10wt% preferably greater than 15%wt% of the total oxygenates present).

Preferably this separation is conducted in two stages, where the mixed alcohol stream is first separated in order to obtain at least one stream comprising C1 to C3 alcohol(s), unreacted synthesis gas and water, and at least one other stream comprising C4 and C4+ alcohol(s); the resulting C4 and C4+ alcohol(s) stream is then preferably subjected to a further separation, in order to obtain a stream comprising C5 and C5+ alcohols and another stream comprising the n-butanols. According to another advantageous embodiment of the present invention, the second alcohols separation stage is performed such that the resulting C4 and C4+ alcohol(s) stream is then preferably subjected to a different separation, in order to obtain a stream comprising C5+ alcohols and another stream comprising the n-butanols and the C5 alcohols which are separated and fed to the dehydration and isomerisation unit of the present invention.; said C5 alcohols will also advantageously dehydrate and isomerised under the same conditions as for the n-butanols, and can thus be respectively converted into branched C5 olefins and then subsequently dimerised to C10 branched materials suitable for gasoline blending.

All of the aforementioned separations may be conducted by any suitable method known to those skilled in the art, for example distillation.

According to a preferred embodiment of this aspect of the present invention, the aforementioned stream(s) comprising C1 to C3 alcohol(s), unreacted synthesis gas and water, recovered in the first stage, is then recycled back into the aforementioned alcohol synthesis unit (e.g. one or more reactors) to undergo further chemical processing. Alternatively, any of the C1 to C3 alcohols present in said stream e.g. methanol, may be isolated, recovered and sold on the market.

According to the present invention, n-butanol is introduced into a conversion reactor and is then dehydrated and isomerised to produce a mixture of olefins, in particular pentene and butene(s) (i.e. 1-butene, 2-butene and iso-butene).

According to one aspect of the present invention at least a part of the said n-butanol introduced into the conversion reactor originates from the aforementioned alcohol synthesis reactor. Alternatively, the alcohol feedstock introduced into the conversion reactor (i.e. n-butanol) may be obtained from a fermentation source, e.g. by the fermentation of, for example, sugars and/or cellulosic materials.

Preferably, when n-butanol is the only feed to the conversion reactor, butene(s) represent together at least 60 wt %, more preferably at least 65 wt% and most preferably at least 70 wt% of the total products exiting the conversion reactor. Preferably, when n-butanol is the only feed to the conversion reactor, pentene represents at least 5 wt % of the total products exiting the conversion reactor. Preferably, the molar the molar ratio of iso-butene:total butenes is at least 0.30, more preferably at least 0.40 and most preferably at least 0.45 .

Furthermore, isobutene represents at least 20 wt%, more preferably at least 30 wt% and most preferably at least 34 wt% of the total products exiting the reactor when the feed to the conversion reactor is n-butanol only. By-products, such as lower and/or higher olefins may also be produced during this dehydration and isomerisation reaction and can optionally be recycled back to the dehydration and isomerisation reactor. If desired, the feedstock entering into the dehydration and isomerisation reactor may be selectively diluted, as necessary, with for example, water or inert gas.

According to the present invention the method for producing the said olefins, e.g. butenes, from the n-butanol containing feedstock is thought to proceed via a combination of:
- dehydrating the said alcohols (e.g. n-butanol) to produce olefins (e.g. n-butene); e.g. 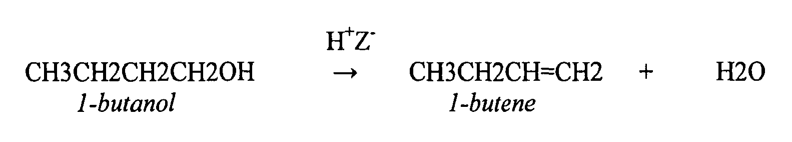 and
- isomerising the olefins thereof (e.g. n-butene) to produce isomers (e.g. iso-butene), e.g. 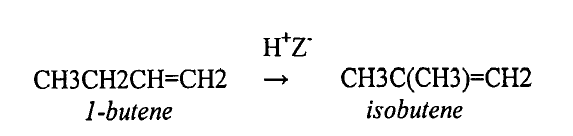 *Where H+Z- is proton exchanged microporous solid with anionic framework Z-.*

Where the said conversion unit (e.g. one or more reactor(s)) is any suitable reactor for dehydrating and isomerising an n-butanol feed, for example a fixed bed, a fluid bed, a slurry reactor or a continuous catalytic distillation reactor (Reactive Distillation).

The said dehydration and isomerisation process is preferably operated at medium to high conversion and at high selectivity.

According to the present invention the catalyst used within the dehydration and isomerisation conversion reactor is a crystalline aluminosilicate (also known as a 'zeolite'), particularly the catalyst used is an acidic zeolitic catalyst and has unidirectional (key structural criterion to achieve high selectivity during the isomerisation stage), non-intersecting channels such as:
- Theta-1(TON-type), with ten-member ring openings (up to 6Å in diameter) with the TON-type structure e.g. aluminosilicates, gallosilicates, zincosilicates, borosilicates, titanosilicates etc. or their germanate counterparts. Preferably, the zeolite-type catalyst is an aluminosilicate TON-type; or
- MTT type zeolites, Ferrierite (FER-type) type zeolites, ZSM-5 and ZSM-23 all with an aluminosilicate composition; or
- SAPO-11 (AEL-type) microporous solids with a silicoaluminophosphate composition.

Where a code consisting of three capital letters has been adopted for each known structure type following the recommendations by IUPAC on zeolite nomenclature ("Chemical Nomenclature and Formulation of Compositions of Synthetic and Natural Zeolites", IUPAC, yellow booklet, 1978).

According to a preferred embodiment of the present invention, the dehydration and isomerisation reaction is carried out in the presence of a Theta-1 porous crystalline aluminosilicate (described in our published European patent specification No. 0057049, which is hereby incorporated by reference). This particular catalyst is produced by mixing together a source of silica, a source of aluminium, a source of alkali metal(s), water and a organic nitrogenous containing base, such as diethanolamine until a homogeneous gel is formed and crystallising the said mixture at a temperature above 70 DEG C for a period of at least 2 hours. Where the said catalyst has the following compositions in terms of the mole ratios of the oxides:

0.9 +/- 0.2 M_{2/n}O : Al₂O₃ : x SiO₂ ; yH₂O

wherein M is at least one cation having a valence n, x is at least 10 and y/x is between 0 and 25, said aluminosilicates having a characteristic X-ray diffraction pattern. The cation M in the aluminosilicates is preferably selected from H⁺, ammonium, alkali metal cations, alkaline earth metal cations, organic nitrogen-containing cations, aluminium cations, gallium cations and mixtures thereof.

The crystalline aluminosilicate used to catalyse the dehydration and isomerisation process is preferably used in the hydrogen form; this can suitably be achieved (in the case of organic containing zeolite) by calcination to remove the organics followed by either ammonium ion exchange followed by further calcination, or by proton exchange with an acid solution; or by a combination thereof. If so desired, the hydrogen form of the zeolite may also be partially or completely exchanged or impregnated with a metal such as Ga or Mg and used in the present process.

The crystalline aluminosilicate used to catalyse the dehydration and isomerisation process may be modified to alter its acidity or shape selectivity in such a way as to improve the catalytic performance. The modifications may include a calcination regime, steam treatment, chemical treatment e.g. with a dealuminating agent, for example, SiCl4, EDTA, aluminating agent e.g. sodium aluminate, AlCl3 etc., inclusion of phosphorous compound, Lewis base, HF etc. A combination of these treatments may also be carried out. The crystalline aluminosilicate may also be treated during the preparation of the hydrogen form or be carried out on the hydrogen-form.

The crystalline aluminosilicate may, if desired, be bound in a suitable binding material either before or after impregnation, or after exchange with a metal compound. The binder may suitably be one of the conventional alumina, silica, clay, or aluminophosphate binders or a combination of binders.

According to a preferred embodiment of the present invention, the dehydration and isomerisation unit is operated at a temperature of more than 200°C, more preferably at a temperature of more than 300°C and most preferably at a temperature of more than 380°C; preferably the temperature of the dehydration and isomerisation unit is less than 650°C, preferably less than 600°C and most preferably less than 500°C.

According to a preferred embodiment of the present invention, the dehydration and isomerisation unit is operated at a pressure of more than 0.01 MPa more preferably of more than 0.05 MPa and most preferably of more than 0.2 MPa; preferably the pressure of the dehydration and isomerisation unit is less than 10 MPa, preferably less than 1 MPa and most preferably less than 1 MPa

According to a preferred embodiment of the present invention, the hourly space velocity (which is defined as the number of moles of n-butanol per litre of catalyst per hour at standard temperature and pressure) may be comprised between 30 and 70.

The optimum hourly space velocity would be dependent on the other operating conditions and the catalyst composition and pretreatment. The relative concentration of the reactants can be optimised by adjusting the olefins to n-butanol ratio. In addition the contact time is optimised at constant hourly space velocity by dilution with inert gas or less reactive gas than the reactants.

The iso-butene dimerisation into di-isobutene reaction is believed to take place at the catalyst pore entrance. Furthermore, the applicants also believe that the 1-butene and 2-butene, produced during the dehydration stage of the process will react with iso-butene (produced during isomerisation) to produce di-isobutene, thus making the dehydration and isomerisation process autocatalytic. Additionally the applicants also believe that 1-butene will react with 2-butene at the pore mouth of the zeolite to produce isobutene (e.g. via the formation of bulky C8 dimers).

The applicants have found an additional advantage by operating the process according to the present invention, in that water is produced as a co-product of the dehydration and isomerisation process. Indeed, this presence of water not only minimises any coke formation but also acts as a diluent.

The resulting olefin stream from the dehydration and isomerisation process is then subjected to a dimerisation procedure, wherein at least part of the olefins produced during dehydration and isomerisation stages are then dimerised in order to produce di-isobutene.

Any prior art isobutene dimerisation process can be used in the process of the present invention. For example, the dimerisation process described in US2005080305 can advantageously be used. This process comprises first oligomerising isobutene to di-isobutene using a sulfonic acid-type ion exchange resin.

According to the present invention the di-isobutene exiting the dimerisation reactor is then recovered e.g. by distillation.

According to a preferred embodiment of the present invention the unconverted olefins (e.g. n-butene) resulting from the dimerisation procedure are then recycled to the dehydration and isomerisation conversion reactor mentioned hereinabove. By performing this embodiment (and thus feeding n-butanol and recycled butenes), the applicants were unexpectedly able to increase the selectivity of the process by reducing the yields of unwanted products, e.g. the C1, C2 and C6 products.

The recycled olefins may be pre-mixed together with the n-butanol feed prior to entering the dehydration and isomerisation reactor, or alternatively they may be fed into the reactor separately and mixed thereafter in the reaction chamber.

The applicants have found it to be advantageous to employ a dimerisation stage, since the separation of the product (i.e. di-isobutene) from the unconverted olefins (such as n-butene) is much more facile than the separation of the iso-butene from the other butenes.

As indicated hereabove, the Applicants have found that selectivity and conversion yield of n-butanols to both iso-butene and pentene was unexpectedly achieved by the process of the present invention. Similarly, the applicants have found if to be advantageous to introduce both the isobutene and the pentene into the dimerisation stage in order to recover a C8-C10 product that can advantageously be sold as a gasoline blend mixture additive. Similarly, the Applicants have also found that the addition of pentanols to the n-butanol feed to the dehydration and isomerisation unit could advantageously be performed according to the present invention.

### EXAMPLE:

The catalyst used is a Theta-1 (TON-type). It was prepared according to the method disclosed in example 1 of EP0485145. It was calcined at 500°C, exchanged with ammonium nitrate solution and then calcined again to convert to the H-form. It was then pressed under 10 ton pressure to form a tablet, which was broken and sieved to form granules between the sizes 0.50 - 0.85mm. Aliquots of 0.50g of the granules were diluted with inert silicon carbide granules of approximately the same size to make up a volume of 7.0 ml. The diluted catalyst was loaded into a tubular reactor (300mm in length and 12 mm internal diameter), sandwiched between beds of inert granules. A flow of air was passed and the catalyst was activated at 550°C overnight. The catalyst temperature was lowered to the value of the catalyst testing and the air flow was replaced with an argon flow. 1-butanol was fed into the catalyst bed via an evaporator at 200°C, through which the gas diluent (argon) was passed, and the argon flow carrying the 1-butanol passed through a line preheated at 200°C before entering the reactor zone. After passing through the catalyst, the product was collected in a condenser kept at room temperature and the gas effluent was them measured and collected for analysis. The liquid product was mostly aqueous with a negligible amount of organic layer. The liquid product was collected and mass balance was calculated. The gas and the aqueous products were analysed off-line by gas chromatography.
The operating temperature was 400°C.
The results are indicated below.

| **Theta-1/TON** | **wt%** |
|---|---|
| trans-butene-2 | 17.35 |
| butene-1 | 10.63 |
| isobutene | 35.94 |
| cis-butene-2 | 12.18 |
| Pentenes | 4.58 |

## Claims

1. Process for the coproduction of pentene and iso-butene by dehydrating and isomerising an n-butanol feedstock in the presence of a crystalline aluminosilicate catalyst to produce pentene and iso-butene.

2. Process for producing di-isobutene and pentene and/or di-pentene from n-butanol, **characterised by** the following consecutive steps:
1) dehydrating and isomerising an n-butanol feedstock in the presence of a crystalline aluminosilicate catalyst to produce an olefin stream comprising pentene, n-butenes and iso-butene,
2) dimerising at least a part of the said stream produced in step 1 to produce a stream comprising di-isobutene and unreacted n-butenes,
3) separating and recycling at least a part of the unreacted n-butenes from step 2 to the dehydration and isomerisation step 1,
4) recovering di-pentene from step 2 and/or separating and recovering pentene from either step 1 and/or step 2, and
5) recovering the di-isobutene from step 2.

3. Process for producing di-isobutene and pentene and/or di-pentene from synthesis gas **characterised by** the following consecutive steps:
1) converting a synthesis gas feed in an alcohol synthesis unit into a mixed alcohol feedstock, containing n-butanol,
2) dehydrating and isomerising a least a part of the n-butanol from step 1, in the presence of a crystalline aluminosilicate catalyst, to produce an olefin stream comprising pentene, n-butenes and iso-butene,
3) dimerising at least a part of the stream produced in step 2 to produce a stream comprising di-isobutene and unreacted n-butenes,
4) separating and recycling at least a part of the unreacted n-butenes from step 3 to the dehydration and isomerisation step 2,
5) recovering di-pentene from step 3 and/or separating and recovering pentene from either step 2 and/or step 3, and
6) recovering the di-isobutene form step 3.

4. Process according to claim 3 wherein the alcohol synthesis unit is operated at a temperature of more than 190 DEG C and less than 400 DEG C; and a pressure of more than 1.9 MPa and less than 20 MPa.

5. Process according to any of claims 3 and 4 wherein the conversion catalyst used in the alcohol synthesis unit is a modified molybdenum sulphide based catalyst, or a modified methanol based catalyst and/or a modified Fischer-Tropsch catalyst and/or a precious metal base catalyst, such as a rhodium based catalyst.

6. Process according to any of claims 3 to 5 wherein the mixed alcohols feedstock containing n-butanol also comprises C1 to C3 alcohol(s) which are separated and recycled into step 1.

7. Process according to any of claims 3 to 6 wherein the mixed alcohols feedstock containing n-butanol also comprises C5 and C5+ alcohols which are separated before the introduction of the n-butanol feedstock into the dehydration and isomerisation stage.

8. Process according to any of claims 3 to 6 wherein the mixed alcohols feedstock containing n-butanol also comprises C5 and C5+ alcohols and wherein the C5+ alcohols are separated before the introduction of a combined n-butanol and C5 alcohol feedstock into the dehydration and isomerisation stage.

9. Process according to any of the preceding claims wherein more than 50%, preferably more than 60%, most preferably more than 70% of the n-butanol is converted into butenes.

10. Process according to any of the preceding claims wherein the molar ratio of iso-butene to total butenes exiting the dehydration and isomerisation stage is at least 0.30, preferably at least 0.40 and most preferably at least 0.45.

11. Process according to any of the preceding claims wherein the crystalline metallosilicate catalyst used for the dehydration and isomerisation stage is an acidic zeolitic catalyst which has unidirectional, non-intersecting channels such as:
- Theta-1 (TON-type), with ten-member ring openings (up to 6Å in diameter) with the TON-type structure e.g. aluminosilicates, gallosilicates, zincosilicates, borosilicates, titanosilicates or their germanate counterparts; or
- MTT type zeolites, Ferrierite (FER-type) type zeolites, ZSM-5 and ZSM-23 all with an aluminosilicate composition; or
- SAPO-11 (AEL-type) microporous solids with a silicoaluminophosphate composition.

12. Process according to claim 11 wherein the catalyst is a Theta-1 porous crystalline aluminosilicate

13. Process according to any of the preceding claims wherein the dehydration and isomerisation stage is performed in a dehydration and isomerisation unit which is operated at a temperature of more than 200°C, more preferably at a temperature of more than 300°C and most preferably at a temperature of more than 380°C; preferably the temperature of the dehydration and isomerisation unit is less than 650°C, preferably less than 600°C and most preferably less than 500°C.

14. Process according to any of the preceding claims wherein the dehydration and isomerisation stage is performed in a dehydration and isomerisation unit which is operated at a pressure of more than 0.01 MPa more preferably of more than 0.05 MPa and most preferably of more than 0.2 MPa; preferably the pressure of the dehydration and isomerisation unit is less than 10 MPa, preferably less than 1 MPa and most preferably less than 1 MPa.

15. Use of a crystalline aluminosilicate catalyst for increasing the yield of conversion of n-butanol into pentene and isobutene during the dehydration and isomerisation stage in a process according to any of the preceding claims.
